Europäisches Patentamt

European Patent Office

Office européen des brevets

(11) Publication number: **0 527 228 A1**

## EUROPEAN PATENT APPLICATION
## published in accordance with Art. 158(3) EPC

(21) Application number: **91904798.5**

(22) Date of filing: **28.02.91**

(86) International application number:
**PCT/JP91/00271**

(87) International publication number:
**WO 92/15398 (17.09.92 92/24)**

(51) Int. Cl.5: **B01J 20/24**

(43) Date of publication of application:
**17.02.93 Bulletin 93/07**

(84) Designated Contracting States:
**CH DE FR GB LI SE**

(71) Applicant: **TOPPAN PRINTING CO. LTD.**
**8-1, Taito 1-chome, Taito-ku**
**Tokyo 110(JP)**

(72) Inventor: **MATSUO, Ryukichi, 20-4,**
**Takagisehigashi 5-chome**
**Saga-shi**
**Saga 849(JP)**
Inventor: **SAISHO, Kenji, 552-4, Nakatsukuma**
**Kitasigeyasumachi Miyaki-gun**
**Saga 849-01(JP)**
Inventor: **MARUTA, Joji, 19-9-601, Kamiogi**
**1-chome**
**Suginami-ku**
**Tokyo 167(JP)**

(74) Representative: **Fiener, Josef**
**Patentanwälte Kahler, Käck & Fiener,**
**Maximilianstrasse 57 Postfach 12 49**
**W-8948 Mindelheim (DE)**

(54) **ATMOSPHERE CONDITIONING AGENT.**

(57) An atmosphere conditioning agent containing a deoxygenating and carbon dioxide generating agent such as ascorbic acid, aiming at improving the adaptability to filling in packaging material, shortening the time required for realizing an anaerobic atmosphere, and regulating the concentration of oxygen and carbon dioxide in an anaerobic atmosphere being conditioned to be each in a desired range. The conditioning agent comprises a carbon dioxide generating agent, a ferrous compound, a pH modifier and water dispersed in a dispersant comprising silicon dioxide and/or calcium silicate, crystalline cellulose, and optionally active carbon. Preferably, according to the gas composition of a desired anaerobic atmosphere, ascorbic acid or calcium carbonate is used as the carbon dioxide generating agent, while calcium hydroxide and/or sodium carbonate is used as the pH modifier.

Rank Xerox (UK) Business Services
(3.10/3.5x/3.0.1)

## TECHNICAL FIELD

The present invention relates to an atmosphere modifier. More particularly, it relates to an atmosphere modifier that can rapidly and simply produce in a closed system an atmosphere having a given oxygen concentration and carbonic acid gas concentration.

## BACKGROUND ART

For the purpose of, e.g., maintaining freshness of food, removing injurious insects from cloths or culturing anaerobic bacteria, the modification of an atmosphere has been hitherto carried out to produce an anaerobic atmosphere by removing oxygen from the atmosphere or by increasing the concentration of carbonic acid gas in the atmosphere.

Various physical or chemical means are known as methods for modifying atmospheres. As a method by which an anaerobic atmosphere in a closed container is safely and simply produced, a method is known in which an oxygen-removing, carbonic acid gas generating material such as ascorbic acid is used (Japanese Patent Publication No. 61-43995). This method makes it possible to control in a closed container of a given capacity, the oxygen concentration to be not more than 0.1 % and also the carbonic acid gas concentration to be within a given range, but it takes a considerable time to achieve such an atmosphere. Thus, it has been sought to shorten the time taken for achieving the desired atmosphere.

For this reason, in order to rapidly achieve the anaerobic atmosphere, an oxygen-removing, carbonic acid gas generating material of a solution type in which sodium ascorbate and a ferrous salt are mixed in water in a specific weight ratio is proposed in relation to the atmosphere modifying method making use of the ascorbic acid or the like (Japanese Patent Application Laid-open No. 61-138533). This oxygen-removing, carbonic acid gas generating material can absorb oxygen at a high rate, but can not readily adjust the absorption of oxygen and the generation of carbonic acid gas. Moreover, the material has been troublesome to handle since it must be prepared right before its use.

Under such circumstances, in order to make it possible for the oxygen gas concentration and carbonic acid gas concentration in a closed container to be rapidly brought to given concentrations and also to make it easier to handle the material, proposals are made on an oxygen-removing, carbonic acid gas generating material of a powder type, comprised of sodium ascorbate, an alkali hydroxide compound, a ferrous salt, calcium silicate, activated carbon, and water separately packaged (Japanese Patent Application Laid-open No. 1-202281), and an oxygen-removing, carbonic acid gas generating material of a powder type in which a solution, a dispersant and a water-absorptive resin are used so that water can be prevented from exuding and the material can be more easily handled (Japanese Patent Application No. 1-224953).

Nonetheless, it has been sought that such oxygen-removing, carbonic acid gas generating materials conventionally used can be much more easily handled when they are packed in packaging materials and also that the anaerobic atmosphere can be more rapidly achieved.

With regard to the anaerobic atmosphere itself also, the conventional oxygen-removing, carbonic acid gas generating materials can only decrease the oxygen concentration merely to 0.1 % or less, or increase the carbonic acid gas concentration to 5 to 10 % or more. In the case of the cultivation of anaerobic bacteria, however, the desired oxygen concentration and carbonic acid gas concentration may vary depending on the types of the bacteria being cultivated. For example, Treponema macrodentium, P. asaccarolyticus, P. magnus, F. nucleatum, E. lentum, C. perfringens, etc. do not grow in an anaerobic atmosphere with an oxygen concentration of 0.5 % or more, but Bacteroides fragilis, etc. grow in an anaerobic atmosphere with an oxygen concentration of 2 to 8 %. Hence, there has been the problem that no optimum anaerobic atmosphere matching the types of the bacteria being cultivated can be produced. As for the cultivation of bacteria, which is carried out usually within the range of 24 hours to 96 hours, the cultivation atmosphere must be maintained for that hours at given oxygen concentration and carbonic acid gas concentration. It, however, has been difficult to simply maintain the cultivation atmosphere to a given state for such a long period of time.

The present invention intends to solve the problems involved in the above conventional oxygen-removing, carbonic acid gas generating materials, and objects thereof are to make it possible to pack an oxygen-removing, carbonic acid gas generating material into a packaging material in an improved suitability and rapidly achieve the anaerobic atmosphere, and also to make it possible to adjust an atmosphere to an anaerobic atmosphere having the desired oxygen concentration and carbonic acid gas concentration.

DISCLOSURE OF THE INVENTION

To achieve the above objects, the present invention provides an atmosphere modifier comprising i) a dispersion medium comprising silicon dioxide and/or calcium silicate, crystal cellulose, and activated carbon optionally used, and ii) dispersed therein a carbonic acid gas generating agent, a ferrous compound, a pH adjuster and water.

Thus, in order to make it easier to handle atmosphere modifiers when they are packed in packaging materials and also to shorten the time taken for achieving the desired atmosphere, the atmosphere modifier of the present invention makes use of a dispersion medium comprising silicon dioxide and/or calcium silicate, crystal cellulose, and activated carbon optionally used.

As a preferred embodiment thereof, ascorbic acid or calcium carbonate is used as the carbonic acid gas generating agent and the pH adjuster is appropriately mixed according to the type of the carbonic acid gas generating agent so that the anaerobic atmosphere having the desired oxygen concentration and carbonic acid gas concentration can be obtained. More specifically, in an instance in which an anaerobic atmosphere with an oxygen concentration of substantially zero and a carbonic acid gas concentration of about 15 % should be obtained, it is preferred to take an embodiment in which ascorbic acid or a salt thereof, a ferrous compound, calcium hydroxide, sodium carbonate and water are dispersed in a dispersion medium comprising silicon dioxide, calcium silicate, crystal cellulose and activated carbon. In an instance in which an anaerobic atmosphere with an oxygen concentration of about 5 % to about 10 % and a carbonic acid gas concentration of about 8 % to about 15 % should be obtained, it is preferred to take an embodiment in which ascorbic acid or a salt thereof, a ferrous compound, sodium carbonate and water are dispersed in a dispersion medium comprising silicon dioxide, calcium silicate, crystal cellulose and activated carbon. In an instance in which an anaerobic atmosphere with an oxygen concentration of about 15 % and a carbonic acid gas concentration of about 8 % to about 10 % should be obtained, it is preferred to take an embodiment in which calcium carbonate, a ferrous compound, calcium hydroxide and water are dispersed in a dispersion medium comprising silicon dioxide and a crystal cellulose.

The atmosphere modifier of the present invention can be prepared by mixing a dispersion medium comprising silicon dioxide and/or calcium silicate and a crystal cellulose, a carbonic acid gas generating agent, a ferrous compound, and a pH adjuster, and then dispersing water in the resulting mixture. The atmosphere modifier of the present invention can also be prepared by mixing a dispersion medium comprising silicon dioxide and/or calcium silicate, crystal cellulose and activated carbon, and a pH adjuster, and then dispersing in the resulting mixture an aqueous solution of a carbonic acid gas generating agent and a ferrous compound.

BEST MODE FOR WORKING THE INVENTION

The present invention will be described below in detail.

In the atmosphere modifier of the present invention, ascorbic acid or a salt thereof, calcium carbonate or the like is used as the carbonic acid gas generating agent, which is selected according to the desired anaerobic atmosphere being achieved. For example, in instances in which an anaerobic atmosphere with an oxygen concentration of substantially zero or about 10 % or less and a carbonic acid gas concentration of about 8 % to about 10 % should be obtained, it is preferred to use a compound such as ascorbic acid or a salt thereof, capable of absorbing oxygen and at the same time generating carbonic acid gas when the compound itself is decomposed. Particularly when the oxygen concentration is made zero and also the carbonic acid gas concentration is controlled to a relatively low concentration, it is preferred to use the ascorbic acid or a salt thereof in a large quantity. Here, the ascorbic acid or a salt thereof may include, for example, L-ascorbic acid, D-iso-ascorbic acid and sodium L-ascorbate. In instances in which an anaerobic atmosphere with an oxygen concentration of about 15 % and a carbonic acid gas concentration of about 8 % to about 10 % should be obtained, it is preferred to use a compound such as calcium carbonate or sodium hydrogencarbonate, capable of generating carbonic acid gas when the compound itself is decomposed.

In the atmosphere modifier of the present invention, ferrous sulfate, ferrous chloride or the like can be used as the ferrous compound used as a reaction catalyst or an agent capable of reacting with the carbonic acid gas generating agent.

The pH adjuster is used so that the reaction to decompose the carbonic acid gas generating agent such as ascorbic acid or calcium carbonate may take place at a given pH value. As the pH adjuster, calcium hydroxide, sodium carbonate or the like can be usually used. Of these, what is actually used is appropriately selected depending on the type of the carbonic acid gas generating agent used and the oxygen concentration or carbonic acid gas concentration in the intended anaerobic atmosphere. For example, in an instance in which the ascorbic acid or a salt thereof is used and an anaerobic atmosphere with an oxygen concentration of substantially zero and a carbonic acid gas concentration of about 8 % to about 15 % should be obtained, it is preferred to use both the calcium hydroxide and sodium carbonate as the pH adjuster. In this instance, the calcium hydroxide has the functions of immediately adjusting the pH value to a value suited for the reaction of decomposing the ascorbic acid or a salt thereof and also absorbing carbonic acid gas produced in excess with respect to a given carbonic acid gas concentration. The sodium carbonate is also presumed to have the function of maintaining a preferred pH value.

In an instance in which the ascorbic acid or a salt thereof is used and an anaerobic atmosphere with an oxygen concentration of about 5 % to about 10 % and a carbonic acid gas concentration of about 8 % to about 15 % should be obtained, it is preferred to use sodium carbonate as the pH adjuster without use of calcium hydroxide. Adjusting the pH with sodium carbonate in this way makes it possible to leave a given quantity of oxygen in the atmosphere. In this instance, increasing the amount of sodium carbonate makes it possible to accelerate the rate at which the anaerobic atmosphere with a given oxygen concentration carbonic acid gas is achieved.

In an instance in which calcium carbonate is used as the carbonic acid gas generating agent and an anaerobic atmosphere with an oxygen concentration of about 15 % and a carbonic acid gas concentration of about 8 % to about 10 % should be obtained, it is possible to use calcium hydroxide.

In the present invention, the dispersion medium components have the functions of bringing the respective chemicals described above used for absorbing oxygen or generating carbonic acid gas into uniform dispersion, securing gas permeability, providing the field of reaction, and also making the product easier to handle when it is packed in a packaging material. Silicon dioxide and/or calcium silicate, crystal cellulose, and activated carbon optionally used are used as the dispersion medium components. Here, the silicon dioxide is used to improve the fluidity of the chemicals after their water retention, and it is preferred to use those having particle diameters of about 10 $\mu$m to about 20 $\mu$m, having a small apparent specific gravity of about 0.1 to about 0.2, having very low water absorption properties, and becoming a little sticky when mixed with an aqueous solution. The calcium silicate is used as a water retainer, and it is preferred to use those having particle diameters of about 20 $\mu$m to about 30 $\mu$m, having an apparent specific gravity of about 0.08 to about 0.12, having water absorption properties about 4 to about 5 times its own weight, and not becoming sticky even when mixed with an aqueous solution. Use of such calcium silicate enables prevention of the damage of the fluidity of chemicals after their water retention. The crystal cellulose is also used as a water retainer, and it is preferred to use those having particle diameters of about 40 $\mu$m to about 200 $\mu$m, having a high apparent specific gravity of about 0.4 to about 0.6, having water absorption properties about one time to about twice its own weight, and becoming sticky when mixed with an aqueous solution. Use of such crystal cellulose can bring about an improvement in the binding properties of the chemicals, can provide appropriate air layers throughout the chemicals because of its high apparent specific gravity, and also can bring about an improvement in fluidity. As for the activated carbon, it is preferred to use a powdery material having a water content of 10 % to 50 %, and particularly about 30 %. Use of such activated carbon makes it easy to mix liquid and powder. Since the activated carbon itself is black, it becomes also possible to visually confirm the state of mixing of the whole chemicals.

Thus, in the present invention, 2 to 4 kinds of components are used as dispersants in combination, whereby the reactivity for oxygen absorption and carbonic acid gas generation and the packing suitability to a packaging material can be improved. In particular, when the particle diameter, specific gravity, water absorption properties of the individual dispersants are set within the above ranges, it becomes possible to improve the reactivity and the packing suitability.

The amounts in which the respective chemicals constituting the atmosphere modifier of the present invention are compounded may be appropriately determined according to the gas composition such as oxygen concentration or carbonic acid gas concentration in the anaerobic atmosphere, the tolerance time for achieving the anaerobic atmosphere, and so forth. In usual instances, the ascorbic acid or a salt thereof and the water may be used in a weight ratio of 0.1:1 to 0.8:1, and preferably 0.4:1 to 0.5:1. An aqueous solution comprising the ascorbic acid or a salt thereof and the dispersion medium may be compounded in a weight ratio of 0.1:1 to 2.0:1. The ascorbic acid or a salt thereof and the ferrous compound may particularly preferably be used in a ratio of 5:2 to 5:3. An aqueous solution of the ascorbic acid or a salt thereof and the ferrous compound may also preferably be in a concentration of 35 % by weight to 45 % by weight.

The atmosphere modifier of the present invention can retain its performance when hermetically closed. Accordingly, as a mode of the product of the present invention on the market, the atmosphere modifier of the present invention may be packed into, e.g., a baglike packaging material having a gas permeability and then the package may be sealed in a hermetic packaging material capable of shutting off oxygen and humidity, which may be unsealed when used.

The atmosphere modifier of the present invention can be prepared by dispersing water to a powdery mixture comprised of the dispersants, carbonic acid gas generating agent, ferrous compound and pH adjuster as previously described. Alternatively, it can be prepared by dispersing an aqueous solution of the carbonic acid gas generating agent and the ferrous compound in a powdery mixture comprised of the dispersants and the pH adjuster. When the powdery mixture and the liquid are mixed, the both may be previously mixed before they are packed in a baglike packaging material. In this instance, the chemicals mixed are so highly reactive that they are inactivated when left in the air. Hence, they must be packed in the state the air is shut off in a nitrogen gas atmosphere. The powdery mixture and the liquid may also be packed simultaneously or successively in a baglike packaging material by means of a two-component packing apparatus, followed by operation such as vibrating, shaking or bending to uniformly mix the powdery mixture and the liquid. In this instance also, they should preferably be packed and mixed in the state the air is shut off in a nitrogen gas atmosphere.

As the packaging material in which the atmosphere modifier of the present invention is packed, it is possible to use, for example, a laminated material the inner surface of which is formed of a film having heat-sealing properties, gas permeability and water resistance and the outer surface of which is formed of a sheet material having a gas permeability.

As the outer-surface material, paper, nonwoven fabric, etc. can be used. These may be coated on their outer surfaces with a water repellent or oil repellent of a silicone type or a fluorine type.

As the inner-surface material, a perforated film such as perforated polyethylene film, or a finely porous film, etc. can be preferably used. As the finely porous film, it is possible to use a film having a Gurley gas permeability rate of 0.01 second to 1,000 seconds/ml, allowing water not to pass at normal pressure, and allowing water vapor to permeate. Commercially available products of such a finely porous film may include DURAGUARD (available from Celanese Chemical Co.), FP-2 (available from Asahi Chemical Co., Ltd.), NF SHEET POLAM (available from Tokuyama Soda Co., Ltd.), and CELLPORE NW01 (available from Sekisui Chemical Co., Ltd.). It is also possible to use mixed paper in which fibers of a thermoplastic resin such as polyethylene are mixed (which has heat-sealing properties and has excellent gas permeability, water resistance, mechanical strength and so forth), nonwoven fabric having heat-sealing properties, nonwoven fabric coated with heat-sealing varnish, waterproof paper coated with heat-sealing varnish, and so forth.

As the hermetic packaging material, a metal foil such as aluminum foil, or a laminate comprising a layer of a resin having excellent gas barrier properties as exemplified by polyvinylidene chloride or a saponified ethylene/vinyl acetate copolymer, can be preferably used. For example, it is possible to use a PET (OPP or Ny)/Al foil/PE(or CPP) laminate or a K-OP(K-PET or K-Ny)/PE( or CPP) laminate. Here, PET represents polyethylene terephthalate; PE, polyethylene; OPP, oriented polypropylene; CPP, casted polypropylene; Ny, polyamide such as nylon; K-OP, oriented polypropylene coated with polyvinylidene chloride; K-PET, polyethylene terephthalate coated with polyvinylidene chloride; and K-Ny, polyamide such as nylon coated with polyvinylidene chloride.

The present invention will be specifically described below by giving Examples. In the following, Examples 1 to 3 and Comparative Example 1 are examples in which oxygen concentration and carbonic acid gas concentration are intended to be 0 % and 9 to 15 %, respectively, in 30 minutes. Examples 4 and 5 and Comparative Examples 2 and 3 are examples in which oxygen concentration and carbonic acid gas concentration are intended to be 5 to 10 % and 9 to 15 %, respectively, in 1 hour. Examples 6 and 7 and Comparative Examples 4 and 5 are examples in which oxygen concentration and carbonic acid gas concentration are intended to be 15 to 21 % and 5 to 10 %, respectively, in 1 hour.

Example 1

In 300 parts by weight of water, 60 parts by weight of ferrous sulfate ($FeSO_4 \cdot 7H_2O$) was dissolved. Next, in the resulting aqueous solution, 120 parts by weight of sodium ascorbate was dissolved. The aqueous solution thus obtained (18 g) and the respective chemicals composed as shown in Table 1 (20 g in total) were compounded to effect uniform dispersion. An atmosphere modifier of the present invention was thus prepared. This was packed in a packaging material (110 mm x 130 mm) comprised of polyethylene mixed paper (50 g/m$^2$). The resulting package was hermetically sealed in a closed container (capacity: 2,500 ml) together with air. Oxygen concentration and carbonic acid gas concentration in the container were measured with time using a zirconia type oxygen analyzer and a light interference type carbonic acid gas analyzer, respectively. Results obtained are shown in Table 2. As shown therein, the oxygen concentration in the container came to be substantially 0 %, and the carbonic acid gas concentration, about 10 %, on the lapse of 30 minutes after the atmosphere modifier of the present invention was put in. The oxygen concentration in the container was maintained at 0 % after 96 hours, and also the carbonic acid gas concentration was maintained at about 12 %.

Example 2

The same aqueous solution (25 g) of sodium ascorbate and ferrous sulfate as used in Example 1 and the respective chemicals composed as shown in Table 1 (30 g in total) were compounded to effect uniform dispersion. An atmosphere modifier of the present invention was thus prepared.

This was packed in a packaging material (90 mm x 120 mm) comprised of polyethylene mixed paper (50 g/m$^2$). In the same manner as in Example 1, the resulting package was hermetically sealed in a closed container together with air, and oxygen concentration and carbonic acid gas concentration in the container were measured with time. Results obtained are shown in Table 2. As shown therein, the oxygen concentration in the container came to be substantially 0 %, and the carbonic acid gas concentration, about 12 %, on the lapse of 30 minutes after the atmosphere modifier of the present invention was put in.

Example 3

The same aqueous solution (18 g) of sodium ascorbate and ferrous sulfate as used in Example 1 and the respective chemicals composed as shown in Table 1 (22 g in total) were compounded to effect uniform dispersion. An atmosphere modifier of the present invention was thus prepared.

This was packed in a packaging material (90 mm x 120 mm) comprised of polyethylene mixed paper (50 g/m$^2$). In the same manner as in Example 1, the resulting package was hermetically sealed in a closed container together with air, and oxygen concentration and carbonic acid gas concentration in the container were measured with time. Results obtained are shown in Table 2. As shown therein, the oxygen concentration in the container came to be substantially 0 %, and the carbonic acid gas concentration, about 9 %, on the lapse of 30 minutes after the atmosphere modifier of the present invention was put in. The oxygen concentration in the container was maintained at 0 % after 96 hours, and also the carbonic acid gas concentration was maintained at 12 %.

Comparative Example 1

The same aqueous solution (18 g) of sodium ascorbate and ferrous sulfate as used in Example 1 and the respective chemicals composed as shown in Table 1 (22 g in total) were compounded to effect uniform dispersion. An atmosphere modifier for comparison was thus prepared.

This was packed in a packaging material (90 mm x 120 mm) comprised of polyethylene mixed paper (50 g/m$^2$). In the same manner as in Example 1, the resulting package was hermetically sealed in a closed container together with air, and oxygen concentration and carbonic acid gas concentration in the container were measured with time. As a result, the rate of oxygen absorption and the rate of carbonic acid gas generation were lower than those in Examples 1 to 3, and the reaction lacked reproducibility. Moreover, the powder showed unsatisfactory dispersibility and fluidity, and was packed in the packaging material with difficulty.

Example 4

The same aqueous solution (6 g) of sodium ascorbate and ferrous sulfate as used in Example 1 and the respective chemicals composed as shown in Table 3 (10 g in total) were compounded to effect uniform dispersion. An atmosphere modifier of the present invention was thus prepared.

This was packed in a packaging material (90 mm x 120 mm) comprised of polyethylene mixed paper (50 g/m$^2$). In the same manner as in Example 1, the resulting package was hermetically sealed in a closed container together with air, and oxygen concentration and carbonic acid gas concentration in the container were measured with time. Results obtained are shown in Table 4. As shown therein, the oxygen concentration in the container came to be 10 % or less, and the carbonic acid gas concentration, about 9 %, on the lapse of 1 hour after the atmosphere modifier of the present invention was put in. The oxygen concentration was about 5 % after 72 hours, and also the carbonic acid gas concentration was about 12 %.

Example 5

The same aqueous solution (6 g) of sodium ascorbate and ferrous sulfate as used in Example 1 and the respective chemicals composed as shown in Table 3 (10 g in total) were compounded to effect uniform dispersion. An atmosphere modifier of the present invention was thus prepared.

This was packed in a packaging material (90 mm x 120 mm) comprised of polyethylene mixed paper (50 g/m$^2$). In the same manner as in Example 1, the resulting package was hermetically sealed in a closed container together with air, and oxygen concentration and carbonic acid gas concentration in the container were measured with time. Results obtained are shown in Table 4. As shown therein, the oxygen concentration in the container came to be 10 % or less, and the carbonic acid gas concentration, about 8 %, on the lapse of 1 hour after the atmosphere modifier of the present invention was put in. The oxygen concentration was about 5 % after 72 hours, and also the carbonic acid gas concentration was about 12 %.

Comparative Example 2

The same aqueous solution (6 g) of sodium ascorbate and ferrous sulfate as used in Example 1 and the respective chemicals composed as shown in Table 3 (10 g in total) were compounded to effect uniform dispersion. An atmosphere modifier for comparison was thus prepared.

This was packed in a packaging material (90 mm x 120 mm) comprised of polyethylene mixed paper (50 g/m$^2$). In the same manner as in Example 1, the resulting package was hermetically sealed in a closed container together with air, and oxygen concentration and carbonic acid gas concentration in the container were measured with time. Results obtained are shown in Table 4. As shown therein, the carbonic acid gas concentration in the container came to be about 9 % or less on the lapse of 1 hour after the atmosphere modifier was put in, but the oxygen concentration did not become 10 % or less. The oxygen concentration was about 5 % after 72 hours, but the carbonic acid gas concentration was more than 15 %.

Comparative Example 3

The same aqueous solution (5 g) of sodium ascorbate and ferrous sulfate as used in Example 1 and the respective chemicals composed as shown in Table 3 (10 g in total) were compounded to effect uniform dispersion. An atmosphere modifier for comparison was thus prepared.

This was packed in a packaging material (90 mm x 120 mm) comprised of polyethylene mixed paper (50 g/m$^2$). In the same manner as in Example 1, the resulting package was hermetically sealed in a closed container together with air, and oxygen concentration and carbonic acid gas concentration in the container were measured with time. Results obtained are shown in Table 4. As shown therein, the carbonic acid gas concentration in the container came to be about 6 % or less on the lapse of 1 hour after the atmosphere modifier was put in, but the oxygen concentration did not become 10 % or less. The oxygen concentration was about 6 % after 72 hours, but the carbonic acid gas concentration was more than 15 %.

Example 6

20 parts by weight of sodium carbonate and 100 parts by weight of ferrous sulfate septahydrate (powder) were compounded with other respective chemicals as shown in Table 5, and 11 g of the resulting compound was further compounded with 2 g of water. An atmosphere modifier of the present invention was thus prepared.

This was packed in a packaging material (80 mm x 100 mm) comprised of nonwoven fabric (50 g/m$^2$) and finely porous polyethylene film (30 $\mu$m thick). In the same manner as in Example 1, the resulting package was hermetically sealed in a closed container together with air, and oxygen concentration and carbonic acid gas concentration in the container were measured with time. Results obtained are shown in Table 6. As shown therein, the oxygen concentration in the container came to be about 18 %, and the carbonic acid gas concentration, about 8 %, on the lapse of 1 hour after the atmosphere modifier of the present invention was put in. The oxygen concentration was about 16 % after 96 hours, and also the carbonic acid gas concentration was maintained at about 8 %.

Example 7

20 parts by weight of sodium carbonate and 100 parts by weight of ferrous sulfate septahydrate (powder) were compounded with other respective chemicals as shown in Table 5, and 10 g of the resulting compound was further compounded with 2 g of water. An atmosphere modifier of the present invention was thus prepared.

This was packed in a packaging material (80 mm x 100 mm) comprised of nonwoven fabric (50 g/m$^2$) and finely porous polyethylene film (30 $\mu$m thick). In the same manner as in Example 1, the resulting package was hermetically sealed in a closed container together with air, and oxygen concentration and carbonic acid gas concentration in the container were measured with time. Results obtained are shown in Table 6. As shown therein, the oxygen concentration in the container came to be about 17 %, and the carbonic acid gas concentration, about 7 %, on the lapse of 1 hour after the atmosphere modifier of the present invention was put in. The oxygen concentration was about 16 % after 96 hours, and also the carbonic acid gas concentration was maintained at about 8 %.

Comparative Example 4

20 parts by weight of sodium carbonate and 75 parts by weight of ferrous sulfate septahydrate (powder) were compounded with other respective chemicals as shown in Table 5, and 10 g of the resulting compound was further compounded with 2 g of water. An atmosphere modifier was thus prepared.

This was packed in a packaging material (80 mm x 100 mm) comprised of nonwoven fabric (50 g/m$^2$) and finely porous polyethylene film (30 $\mu$m thick). In the same manner as in Example 1, the resulting package was hermetically sealed in a closed container together with air, and oxygen concentration and carbonic acid gas concentration in the container were measured with time. Results obtained are shown in Table 6. As shown therein, the oxygen concentration did not come to be about 17 %, and the carbonic acid gas concentration, about 5 %, before the lapse of about 24 hours after the atmosphere modifier was put in.

Comparative Example 5

20 parts by weight of sodium carbonate and 75 parts by weight of ferrous sulfate septahydrate (powder) were compounded with other respective chemicals as shown in Table 5, and 10 g of the resulting compound was further compounded with 2 g of water. An atmosphere modifier was thus prepared.

This was packed in a packaging material (80 mm x 100 mm) comprised of nonwoven fabric (50 g/m$^2$) and finely porous polyethylene film (30 $\mu$m thick). In the same manner as in Example 1, the resulting package was hermetically sealed in a closed container together with air, and oxygen concentration and carbonic acid gas concentration in the container were measured with time. Results obtained are shown in Table 6. As shown therein, the oxygen concentration did not come to be about 17 %, and the carbonic acid gas concentration, about 5 %, before the lapse of about 24 hours after the atmosphere modifier was put in.

Table 1

(parts by weight)

|  | Example 1 | Example 2 | Example 3 | Comparative Example 1 |
|---|---|---|---|---|
| Dispersion medium: |  |  |  |  |
| MF | 25 | 25 | 20 | 57 |
| FR | 15 | 15 | 15 | 14 |
| TG | 20 | 20 | 20 | − |
| W-30 | 20 | 20 | 20 | 14 |
| IH | − | − | − | 9 |
| Calcium hydroxide | 8 | 8 | 10 | 6 |
| Sodium carbonate | 12 | 12 | 10 | − |

(Remarks)

MF: Silicon dioxide; trade name: MYCON-F; available from Tomita Seiyaku KK.

FR: Calcium silicate; trade name: FLOWRYTE-R; available from Tokuyama Soda Co., Ltd.

TG: Crystal cellulose; trade name: ABICEL; available from Asahi Chemical Co., Ltd.

W-30: Activated carbon; trade name: CARBORAFIN; available from Takeda Chemical Industries, Ltd.

IH: Water-absorptive resin; trade name: DIAWET; available from Mitsubishi Petrochemical Company Limited.

Table 2

|  | $O_2/CO_2$ concentration (%) | | | | |
|---|---|---|---|---|---|
|  | 10 min. | 20 min. | 30 min. | 40 min. | 96 hr. |
| Example: | | | | | |
| 1 | 2.1/9.9 | 0.37/10.5 | 0.05/10.6 | 0/11.0 | 0/12.3 |
| 2 | 1.4/11.5 | 0.16/12.4 | 0.01/12 | 0/11.8 | – |
| 3 | 2.8/5.5 | 0.9/7.5 | 0.18/9.1 | 0.07/9.1 | 0/12.0 |

Table 3

(parts by weight)

|  | Example 4 | Example 5 | Comparative Example 2 | Comparative Example 3 |
|---|---|---|---|---|
| Dispersion medium: | | | | |
| MF | 20 | 20 | 20 | 30 |
| FR | 15 | 20 | 20 | 20 |
| TG | 20 | 20 | – | – |
| W–30 | 20 | 10 | 20 | 20 |
| IH | – | – | 20 | 20 |
| Calcium hydroxide | – | – | – | – |
| Sodium carbonate | 25 | 30 | 20 | 10 |

Table 4

| | $O_2/CO_2$ concentration (%) | | | | |
|---|---|---|---|---|---|
| 1 hr. | 2.5 hr. | 4 hr. | 24 hr. | 48 hr. | 96 hr. |
| **Example 4:** | | | | | |
| 8.5/9.0 | 8.1/8.2 | 7.8/8.9 | 6.3/10.8 | – | 5.1/12.4 |
| **Example 5:** | | | | | |
| 8.5/8.3 | 8.3/8.3 | 7.6/8.9 | 6.5/10.5 | 5.8/11.4 | 5.3/11.9 |
| **Comparative Example 2:** | | | | | |
| 10.4/9.9 | 9.5/11.3 | 8.2/13.2 | 6.4/16.0 | 5.4/17.1 | 5.0/17.0 |
| **Comparative Example 3:** | | | | | |
| 13.9/5.5 | 11.9/8.4 | 11.0/8.7 | 7.5/13.0 | 6.8/14.5 | 6.1/15.1 |

Table 5

(parts by weight)

| | Example 6 | Example 7 | Comparative Example 4 | Comparative Example 5 |
|---|---|---|---|---|
| **Dispersion medium:** | | | | |
| MF | 20 | 20 | – | 50 |
| FR | – | – | – | – |
| TG | 50 | 50 | 100 | |
| W-30 | 15 | – | – | – |
| IH | – | – | – | – |
| Calcium hydroxide | 5 | 10 | 10 | 10 |
| Calcium carbonate | 20 | 20 | 15 | 15 |
| Ferrous sulfate septahydrate | 100 | 100 | 75 | 75 |

11

Table 6

| | $O_2/CO_2$ concentration (%) | | | | | |
|---|---|---|---|---|---|---|
| | 1 hr. | 2 hr. | 4 hr. | 24 hr. | 48 hr. | 96 hr. |
| Example 6: | | | | | | |
| | 18.6/6.0 | 17.5/6.3 | 17.0/7.4 | 16.5/8.4 | − | 16.3/8.5 |
| Example 7: | | | | | | |
| | 17.0/7.3 | 16.6/7.8 | 16.6/7.8 | 16.2/7.6 | − | 16.5/8.0 |
| Comparative Example 4: | | | | | | |
| | − | − | − | 17.4/5.2 | 17.3/5.2 | 17.0/4.8 |
| Comparative Example 5: | | | | | | |
| | − | − | − | 17.6/4.6 | 17.4/5.0 | 17.0/4.6 |

INDUSTRIAL UTILIZATION

The atmosphere modifier of the present invention brings about an improvement in the packing suitability to packaging materials to make it easy to handle, and can rapidly change an atmosphere in a given closed container to an anaerobic atmosphere. It becomes also possible to adjust an atmosphere to an anaerobic atmosphere having the desired oxygen concentration and carbonic acid gas concentration.

**Claims**

1. An atmosphere modifier comprising i) a dispersion medium comprising silicon dioxide and/or calcium silicate and crystal cellulose, and ii) dispersed therein a carbonic acid gas generating agent, a ferrous compound, a pH adjuster and water.

2. The atmosphere modifier according to Claim 1, wherein said dispersion medium further comprises activated carbon.

3. The atmosphere modifier according to Claim 2, wherein said carbonic acid gas generating agent comprises ascorbic acid or a salt thereof.

4. The atmosphere modifier according to Claim 1 or 2, wherein said carbonic acid gas generating agent comprises calcium carbonate.

5. The atmosphere modifier according to any one of Claims 1 to 4, wherein said pH adjuster comprises calcium hydroxide and/or sodium carbonate.

6. The atmosphere modifier according to Claim 2, wherein said dispersion medium comprises silicon dioxide, calcium silicate, crystal cellulose and activated carbon, said carbonic acid gas generating agent comprises ascorbic acid or a salt thereof, and said pH adjuster comprises calcium hydroxide and sodium carbonate.

EP 0 527 228 A1

7. The atmosphere modifier according to Claim 2, wherein said dispersion medium comprises silicon dioxide, calcium silicate, crystal cellulose and activated carbon, said carbonic acid gas generating agent comprises ascorbic acid or a salt thereof, and said pH adjuster comprises sodium carbonate.

8. The atmosphere modifier according to Claim 1, wherein said dispersion medium comprises silicon dioxide and crystal cellulose, and said pH adjuster comprises calcium hydroxide.

9. The atmosphere modifier according to Claim 8, wherein said dispersion medium further comprises activated carbon.

10. A method of preparing the atmosphere modifier as described in Claim 1, comprising mixing a dispersion medium comprising silicon dioxide and/or calcium silicate and crystal cellulose, a carbonic acid gas generating agent, a ferrous compound, a pH adjuster and water to give a mixture, and dispersing water in said mixture.

11. A method of preparing the atmosphere modifier as described in Claim 2, comprising mixing a dispersion medium comprising silicon dioxide and/or calcium silicate, crystal cellulose and activated carbon, and a pH adjuster to give a mixture, and dispersing in said mixture an aqueous solution of a carbonic acid gas generating agent and a ferrous compound.

12. A method of preparing the atmosphere modifier as described in Claim 6, comprising mixing a dispersion medium comprising silicon dioxide, calcium silicate, crystal cellulose and activated carbon, calcium hydroxide, and sodium carbonate to give a mixture, and dispersing in said mixture an aqueous solution of ascorbic acid or a salt thereof and a ferrous compound.

13. A method of preparing the atmosphere modifier as described in Claim 7, comprising mixing a dispersion medium comprising silicon dioxide, calcium silicate, crystal cellulose and activated carbon, and sodium carbonate to give a mixture, and dispersing in said mixture an aqueous solution of ascorbic acid or a salt thereof and a ferrous compound.

14. A method of preparing the atmosphere modifier as described in Claim 8, comprising mixing a dispersion medium comprising silicon dioxide and crystal cellulose, calcium carbonate, a ferrous compound, and calcium hydroxide to give a mixture, and dispersing water in said mixture.

15. The method of preparing the atmosphere modifier according to Claim 14, wherein said dispersion medium further comprises activated carbon.

13

# INTERNATIONAL SEARCH REPORT

International Application No PCT/JP91/00271

**I. CLASSIFICATION OF SUBJECT MATTER** (if several classification symbols apply, indicate all) [6]

According to International Patent Classification (IPC) or to both National Classification and IPC

Int. Cl[5] B01J20/24

**II. FIELDS SEARCHED**

| Minimum Documentation Searched [7] | |
|---|---|
| Classification System | Classification Symbols |
| IPC | B01J20/00-20/24 |

Documentation Searched other than Minimum Documentation
to the Extent that such Documents are Included in the Fields Searched [8]

| | |
|---|---|
| Jitsuyo Shinan Koho | 1926 - 1991 |
| Kokai Jitsuyo Shinan Koho | 1971 - 1991 |

**III. DOCUMENTS CONSIDERED TO BE RELEVANT** [9]

| Category* | Citation of Document, [11] with indication, where appropriate, of the relevant passages [12] | Relevant to Claim No. [13] |
|---|---|---|
| Y | JP, A, 1-202281 (Toppan Printing Co., Ltd.), August 15, 1989 (15. 08. 89), (Family: none) | 1-15 |
| Y | JP, U, 60-128720 (Toppan Printing Co., Ltd.), August 29, 1985 (29. 08. 85), (Family: none) | 1-15 |
| Y | JP, A, 2-178337 (Tokuyama Soda Co., Ltd.), July 11, 1990 (11. 07. 90), (Family: none) | 1-15 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| May 14, 1991 (14. 05. 91) | May 27, 1991 (27. 05. 91) |
| International Searching Authority | Signature of Authorized Officer |
| Japanese Patent Office | |

Form PCT/ISA/210 (second sheet) (January 1985)